(19) Europäisches Patentamt
European Patent Office
Office européen des brevets

(11) **EP 4 261 842 A1**

(12) **EUROPEAN PATENT APPLICATION**

(43) Date of publication:
**18.10.2023 Bulletin 2023/42**

(21) Application number: **22187566.9**

(22) Date of filing: **28.07.2022**

(51) International Patent Classification (IPC):
**G16H 50/20** (2018.01)    **G16H 50/30** (2018.01)

(52) Cooperative Patent Classification (CPC):
**G16H 50/20; G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.04.2022 US 202263331277 P**

(71) Applicant: **Koninklijke Philips N.V.**
**5656 AG Eindhoven (NL)**

(72) Inventors:
• **HOLDRICH SCHWAGER, Emma**
**Eindhoven (NL)**
• **GHOSH, Erina**
**Eindhoven (NL)**

(74) Representative: **Philips Intellectual Property & Standards**
**High Tech Campus 52**
**5656 AG Eindhoven (NL)**

(54) **APPARATUS AND METHOD FOR GENERATING AN ALERT**

(57)    Provided is an apparatus and method for combining the outputs of separate predictive models or algorithms to generate a single alert. The apparatus includes a framework which uses rules to determine a single alert to be generated across all the predictive models or algorithms based on evaluating each model separately at each timepoint based on their model scores. Using the proposed rules, the results of each of the models are combined to generate a single alert at each timepoint or window. In case of conflicting results from the models, the generation of the alert is suppressed.

EP 4 261 842 A1

Fig. 1

**Description**

TECHNICAL FIELD

The present disclosure generally relates to patient health monitoring and generating alerts related to a condition of a patient. More particularly, embodiments herein relate to combining outputs of multiple predictive models or algorithms to generate a single alert pertaining to a condition of a patient.

BACKGROUND

Multiple models, algorithms, or risk scores have been developed that may predict different facets of a single problem to increase their utility in a clinical set up. This can be for a single outcome or multiple related outcomes.

For example, erstwhile solutions separately predict the risk of disease onset and disease progression for complications such as, but not limited to, Acute Respiratory Distress Syndrome (ARDS) or Acute Kidney Injury (AKI). On the other hand, it may also be possible to predict different but related complications, such as, for instance, separately predicting ventilator-associated pneumonia (VAP) and barotrauma in invasively mechanically ventilated patients.

By predicting multiple facets of the problem, more information is available about the precise state and prognosis of the patient.

For example, a clinician can determine not just whether the patient is likely to have ARDS or AKI, but whether it will likely be severe. It remains an open question how faceted models such as these may be integrated into the clinical workflow.

Current examples of clinical decision support (CDS) in the marketplace are limited to simple predictive algorithms such as, for example, predicting the onset of sepsis or hemodynamic instability. Even these simple models have a great variety in terms of the interfaces used by clinicians.

Furthermore, more-complex modeling frameworks have not been commercially explored.

Having multiple models can increase clinical utility, but also lead to generation of conflicting information. Further, having multiple models with separate alerts can increase alarm fatigue or information overload.

Limitations and disadvantages of conventional and traditional approaches will become apparent to one of ordinary skill in the art, through comparison of described systems with some aspects of the present disclosure, as set forth in the remainder of the present application and with reference to the drawings.

SUMMARY

As discussed above, it is currently challenging to use output of multiple predictive models as a reliable source for influencing a clinical outcome or decision. A claimed solution rooted in computer technology overcomes problems specifically arising in the realm of computer technology when processing predictive outputs from multiple different types of predictive models or algorithms.

In the claimed solution of the present disclosure, a framework is described that combines the outputs from multiple types of predictive models to generate a single alert to trigger clinical action protocols. Such a framework may be used for any clinical decision support algorithms or models that may be used to predict potential risk of occurrence of a health condition at varying levels of severity in CDS systems. The health condition may include, but is not limited to, Acute Respiratory Distress Syndrome (ARDS), Acute Decompensated Heart Failure (ADHF), Hemodynamic Instability and Acute Kidney Injury (AKI).

In some implementations herein, the framework comprises a user interface communicatively coupled to a processing system. The framework receives outputs corresponding to each of a plurality of predictive models. These predictive models are trained for predicting potential risk of occurrence of a health condition at varying levels of severity in CDS systems. In some embodiments, each of the predictive models may also run their independent CDS algorithms.

The framework also receives the model scores pertaining to each predictive model. The framework then combines the outputs of the plurality of predictive models to generate an alert at a given time (a timepoint or a window), based on one or more rules and the model scores received for each of the plurality of predictive models.

The processing system of the framework is configured to compare the model scores received for each of the plurality of predictive models with one or more model-specific thresholds at the given time, to obtain a set of alert trigger signals. The processing system is further configured to determine if at least one alert trigger signal in the set of alert trigger signals is conflicting with at least one other alert trigger signal in the set of alert trigger signals based on the one or more rules. In response to a determination that the at least one alert trigger signal conflicts with the at least one other alert trigger signal, the processing system is configured to suppress generation of the alert.

A principal object of the present disclosure is to deal with conflicting results or conflicting predictions from multiple predictive models by combining their outputs to provide reliable real-time decisions.

Another object of the present disclosure is the provision of a model-agnostic framework that may be adapted to receive model scores and process triggers from different types of predictive models, irrespective of the decision algorithms running on such models or the training mechanism implemented for such models.

A further object of the present disclosure is to generate a single alert based on processing triggers from multiple types of predictive models using a set of rules. The set of rules enables effectively processing the triggers at every timepoint or window. At each timepoint or window, the model scores are compared with the model-specific thresholds to generate 'triggers'. Finally, the triggers from each model are combined to generate a single alert. Thus, the present disclosure provides the advantage of redefining triggers at every timepoint or window, to provide reliable, accurate decision results.

Further, by providing an apparatus that can suppress generation of the alert when there are conflicting triggers, contradictory predictions from the predictive models may be managed in an effective manner, thus preventing mistrust of the models.

Furthermore, the present disclosure provides the advantage of generating only a single alert for effective decision making, instead of overburdening users such as clinicians with multiple alerts that may be cumbersome to manage and that which may be unreliable, leading to indecisive outcomes.

In one aspect, the processing system is configured to combine the outputs of the plurality of predictive models to generate an alert based on single timepoint-based rules. In response to a determination that the at least one alert trigger signal does not conflict with the at least one other alert trigger signal, the processing system is configured to select at each timepoint, an alert trigger signal from the set of alert trigger signals based on a severity level of the alert trigger signal and generate the alert based on the selected alert trigger signal.

Consider for instance, a two-model predictive system including an any-disease predictive model and a severe-disease predictive model. Upon determining if the any-disease predictive model and the severe-disease predictive model both generate alert trigger signals, the processing system is configured to issue an alert corresponding to a most-severe alert trigger signal.

Upon determining if the severe-disease predictive model generates an alert trigger signal but the any-disease predictive model does not generate an alert trigger signal, the processing system is configured to suppress issuance of an alert.

In another aspect, the processing system is configured to combine the outputs of the plurality of predictive models to generate an alert based on a prioritization matrix. The prioritization matrix is an M x M matrix of weights given to different combinations of the plurality of predictive models. The processing system is configured to generate an alert weight vector comprising alert weights corresponding to each of the predictive models by multiplying an alert trigger signal vector by the prioritization matrix. The alert trigger signal vector comprises alert trigger signals from each of the predictive models.

The processing system is configured to suppress generation of the alert if at least one of the alert weights in the alert weight vector is less than zero. An alert is generated corresponding to a predictive model with a maximum alert weight in the alert weight vector.

Consider for instance, a two-model predictive system comprising an any-disease predictive model and a moderate/severe-disease predictive model. Upon determining if the any-disease predictive model generates an alert trigger signal and the moderate/severe-disease predictive model does not generate any alert trigger signal, the processing system is configured to issue an alert corresponding to the any-disease predictive model.

Upon determining if the any-disease predictive model does not generate any alert trigger signal and the moderate/severe-disease predictive model generates an alert trigger signal, the processing system is configured to suppress issuance of an alert due to the contradiction in the determination.

Upon determining if the any-disease predictive model and the moderate/severe-disease predictive model both generate alert trigger signals, the processing system is configured to issue an alert corresponding to the moderate/severe-disease predictive model.

In yet another aspect, the processing system is configured to combine the outputs of the plurality of predictive models to generate an alert based on windowing-based rules. In response to a determination that the at least one alert trigger signal does not conflict with the at least one other alert trigger signal, the processing system is configured to select at each window, an alert trigger signal from the set of alert trigger signals based on a severity level of the alert trigger signal, and generate the alert based on the selected alert trigger signal.

Consider for instance, a two-model predictive system comprising an any-disease predictive model and a severe-disease predictive model. Upon determining if the any-disease predictive model and the severe-disease predictive model both generate alert trigger signals, the processing system is configured to issue an alert corresponding to a most-severe alert trigger signal.

Upon determining the severe-disease predictive model generates an alert trigger signal but the any-disease predictive model does not generate an alert trigger signal, the processing system is configured to suppress issuance of an alert.

It should be appreciated that all combinations of the foregoing concepts and additional concepts discussed in greater detail below (provided such concepts are not mutually inconsistent) are contemplated as being part of the subject matter disclosed herein. In particular, all combinations of claimed subject matter appearing at the end of this disclosure are contemplated as being part of the subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

## BRIEF DESCRIPTION OF THE DRAWINGS

The various advantages of the embodiments will become apparent to one skilled in the art by reading the following specification and appended claims, and by referencing the following drawings, in which:

FIG. 1 is a block diagram illustrating an example system implementing systems and/or methods described herein in accordance with an exemplary embodiment of the disclosure.

FIG. 2 is a block diagram illustrating an alert generation apparatus in accordance with an exemplary embodiment of the disclosure.

FIG. 3 is a schematic illustrating how scores from two models can be combined using the proposed rules in accordance with an exemplary embodiment of the disclosure.

FIG. 4 is a flowchart illustrating a method for generating an alert in accordance with an exemplary embodiment of the disclosure.

FIG. 5 is a flowchart illustrating a method for combining the outputs of the plurality of predictive models to generate an alert in accordance with an exemplary embodiment of the disclosure.

FIG. 6 is a flowchart illustrating a method for combining outputs of predictive models using single timepoint-based rules in accordance with an exemplary embodiment of the disclosure.

FIG. 7 is a flowchart illustrating a method for combining outputs of predictive models using a prioritization matrix in accordance with an exemplary embodiment of the disclosure.

FIG. 8 is a flowchart illustrating a method for combining outputs of predictive models using windowing-based rules in accordance with an exemplary embodiment of the disclosure.

FIG. 9 is a block diagram illustrating an example computer program product in accordance with an exemplary embodiment of the disclosure.

FIG. 10 is a diagram illustrating an example of the alert generation apparatus in accordance with an exemplary embodiment of the disclosure.

FIG. 11 is a diagram illustrating a semiconductor apparatus in accordance with an exemplary embodiment of the disclosure.

## DETAILED DESCRIPTION

As will be described in greater detail below, in some implementations discussed herein, a framework may advantageously be used to address parts of the challenges in using multiple predictive models as a reliable source for real-time decisions.

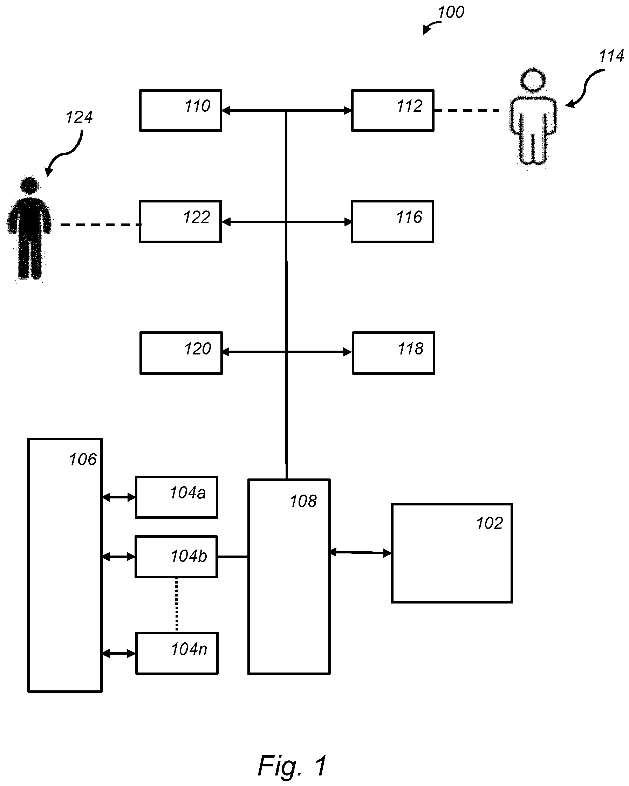

FIG. 1 is a block diagram illustrating an example system implementing systems and/or methods described herein in accordance with an exemplary embodiment of the disclosure. Referring to FIG. 1, there is shown a health management system 100 that includes an alert generation apparatus 102, a plurality of predictive models 104a-104n, a main platform 106, a decision support application 108, a patient monitor 110, sensor(s) 112, a patient 114, a therapeutic device 116, a medical management device 118, a database 120, a user interface 122, user interface(s) 122 and a user 124.

The health management system 100 may be centralized or may be distributed and may include some or all elements and components of one or more computers or computer systems.

In some implementations, the health management system 100 may be utilized as a control point element of an Integrated Clinical Environment (ICE). As used herein, the "Integrated Clinical Environment (ICE)" refers to a platform to create a medical Internet of Things (IoT) associated with the care of a patient. In such an implementation, the health management system 100 may support many real-time clinical decision support algorithms and closed loop control algorithms of medical devices in the ICE.

In the illustrated implementation, the health management system 100 may include the main platform 106. In some implementations, the main platform 106 may be embodied as a server computer or a plurality of server computers (e.g., interconnected to form a server cluster, cloud computing resource, the like, and/or combinations thereof).

In some implementations, the plurality of predictive models 104a-104n may be associated with the main platform 106 and may each include the decision support application 108. The operations of the decision support application 108 trigger notifications and/or clinical interventions related to different health conditions that may include, but are not limited to, Acute Respiratory Distress Syndrome (ARDS), Acute Decompensated Heart Failure (ADHF), Hemodynamic Instability and Acute Kidney Injury (AKI).

In some implementations, the alert generation apparatus 102 may be communicatively coupled with the main platform 106. The alert generation apparatus 102 combines the outputs or triggers from the plurality of predictive models 104a-104n to generate a single alert based on a set of rules. The rules may include, but are not limited to, single timepoint-based rules, a prioritization matrix, and windowing-based rules.

As will be described in further detail in conjunction with different figures and embodiments, several different techniques of combining the outputs or triggers of the plurality of predictive models 104a-104n for generating a single alert for clinical interventions using the alert generation apparatus 102 may be implemented in the health management system 100.

Additionally, or alternatively, the health management system 100 may include the patient monitor 110, the sensor(s) 112 that that may be associated with the patient 114, the therapeutic device 116, the medical management device 118, the database 120, the user interface(s) 122 that may be associated with the user 124, the like, and/or combinations thereof. For instance, the main platform 106, the patient monitor 110, the sensor(s) 112, the therapeutic device 116, the medical management device 118, the database 120, and/or the user interface(s) 122 may be in communication with one another via communication technologies that may include, but are not limited to, Internet-based communicating, cloud-based communication, wired communication, wireless communication, the like, and/or combinations thereof.

In an example, the patient monitor 110 may be utilized to enter patient data to the database 120. For example, the patient monitor 110 may determine patient data measured via the sensor(s)112. In such an example, the patient monitor 110 may be configured to monitor a patient for vital signs and the like, and the patient monitor 110 may communicate such measured patient data to the database 120. For instance, the sensor(s) 112 may determine dynamic patient condition data including patient vitals that may include, but are not limited to, blood pressure, pulse, temperature, respiration, and/or the like, and/or patient test results that may include, but are not limited to, creatine level, urine flow, potassium level, oxygen saturation, blood glucose level, carbon dioxide level, and/or the like.

In some implementations, the patient monitor 110 may comprise a bedside-type monitor, a transport-type monitor, a central station-type monitor, the like, and/or combinations thereof.

In some implementations, the sensor(s) 112 may be minimally invasive-style sensors (e.g., by puncturing the skin, sensing through the skin, the like, and/or combinations thereof). Sensor(s) 112 may be wired or wireless. In implementations where the sensor(s) 112 are wireless, the sensors 112 may have power consumption limitations. Accordingly, operations to increase the patient monitoring frequency (e.g., by increasing data collection frequency and/or data transmission frequency) typically will adversely impact power consumption. Accordingly, it is advantageous to increase the patient monitoring frequency in a limited manner responsive to the needs of each individual patient.

In another example, the therapeutic device 116 may be utilized to enter patient data to the database 120. For example, the therapeutic device 116 may determine measured patient data. In such an example, the therapeutic device 116 may be configured to monitor the delivery of a particular therapy (e.g., a non-medication treatment) to a patient and may communicate such measured patient data to database 120.

In some implementations, the therapeutic device 116 may supply and/or monitor the administration of one or more patient procedures (e.g., dialysis).

In a further example, the medical management device 118 may be utilized to enter patient data to the database 120. For example, the medical management device 118 may determine measured patient data. In such an example, the medical management device 118 may be configured to monitor medication delivery to a patient and may communicate such measured patient data to the database 120.

In some implementations, the medical management device 118 may supply and/or monitor the administration of one or more patient medications (e.g., blood pressure medications, diuretic medications, anti-anemia medications, cholesterol lowering medications, vitamin supplements, and/or the like).

Additionally, or alternatively, in a still further example, the user interface 122 may be utilized to enter patient data to the database 120. In some implementations, user interface 122 may be implemented via one or more formfactor devices (e.g., a smart phone, a tablet, a laptop, a workstation, and/or the like), an interface associated with the main platform 106, and/or an interface associated with the patient monitor 110. Additionally, or alternatively, a care provider (e.g., the user 124) may enter measured patient data through an analog device, a non-networked patient monitor, a non-networked therapeutic device, a non-networked medical management device, the like, and/or combinations thereof. In such an example, the care provider may enter such measured patient data to the database 120 via the user interface(s) 122.

In the illustrated implementation, the database 120 may include one or more types of patient data. For example, the database 120 may include patient data including laboratory result data, microbiology data, medication data, vital sign data, care order data, admission discharge and transfer data, and/or the like. As used herein, the term "database" refers to a collection of data and information organized in such a way as to allow the data and information to be stored, retrieved, updated, and/or manipulated. The term "database" as used herein may also refer to databases that may reside locally or that may be accessed from a remote location (e.g., via remote network servers).

As used herein, the term "patient data " refers to data or information for identifying an individual. Patient data may include measured patient data from an analog medical device, a sensor, a patient monitor, a therapeutic device, a medical management device, a medical imaging device, the like, and/or combinations thereof. Additionally, patient data may include a patient's name, age, weight, previous medical history, admission number, medical personnel in charge, date of admission, medical condition, a medical status, the like, and/or combinations thereof.

Additionally, or alternatively, in some implementations, the database 120 may include or be associated with a simulated database. In such an example, such a simulated database may generate estimated patient data. For example, the simulated database may utilize some measured patient data from the patient monitor 110, the sensor(s) 112, the therapeutic device 116, the medical management device 118, and/or the user interface(s) 122 to generate some other estimated patient data. Such a simulated database may utilize digital twin technology to perform the estimation, for example. In such an example, such estimated patient data may be marked to indicate its estimated nature (rather than measured patient data). Additionally, or alternatively, a weight factor may be applied to the estimated patient data so that the estimated patient data may have a lower weight than corresponding measured patient data.

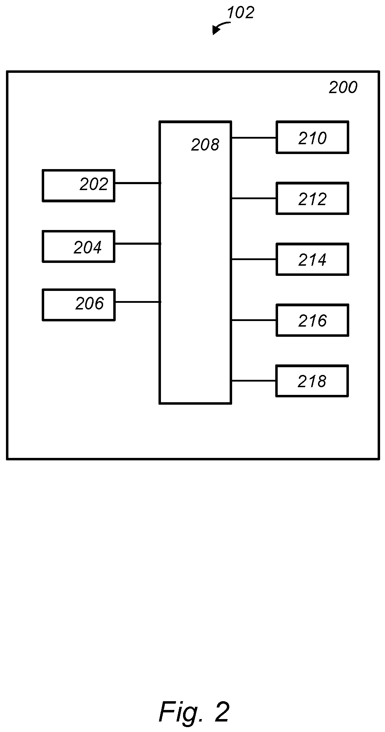

FIG. 2 is a block diagram illustrating an alert generation apparatus in accordance with an exemplary embodiment of the disclosure. Referring to FIG. 2, there is shown the alert generation apparatus 102 that includes a framework 200 that further includes a memory 202, a user interface 204, a processing system 206, a communications unit 208, a receiving component 210, a model scores comparison component 212, a rules component 214, a conflict evaluation component 216 and an alert generator 218.

The memory 202 may include, but is not limited to, one or more memory devices, persistent storage, computer-readable storage media, random access memory (RAM) and cache memory. In general, the memory 202 may include any suitable volatile or non-volatile computer-readable storage media. The memory 202 may comprise suitable logic, and/or interfaces, that may be configured to store instructions (for example, computer readable program code) that can implement various aspects of the present disclosure.

The memory 202 is communicatively coupled to the user interface 204 and the processing system 206.

The user interface 204 may be implemented via one or more form-factor devices that may include, but are not limited to, a smart phone, a tablet, a laptop, a workstation, and/or the like.

The processing system 206 may comprise suitable logic, interfaces, and/or code that may be configured to execute the instructions stored in the memory 202 to implement various functionalities of the alert generation apparatus 102 in accordance with various aspects of the present disclosure. The processing system 206 may be further configured to communicate with various modules of the alert generation apparatus 102 via the communications unit 208.

The communications unit 208 may be configured to transmit data between modules, engines, databases, memories, and other components of the alert generation apparatus 102 for use in performing the functions discussed herein. The communications unit 208 may include one or more communication types and utilize various communication methods for communication within the alert generation apparatus 102.

The processing system 206, in some implementations, includes the receiving component 210, the model scores comparison component 212, the rules component 214, the conflict evaluation component 216 and the alert generator 218.

The receiving component 210 may comprise suitable logic, interfaces, and/or code that may be configured to receive outputs of each predictive model of the plurality of predictive models 104a-104n. In some embodiments, the plurality of predictive models 104a-104n are trained for predicting potential risk of occurrence of a health condition at varying levels of severity in CDS systems. Each predictive model may run its own CDS algorithm to predict the occurrence of a health condition to aid clinical decisions or intervention protocols.

Further, the receiving component 210 is configured to receive model scores corresponding to each predictive model of the plurality of predictive models 104a-104n. In some implementations, the model scores are input by a user via the user interface 204.

The processing system 206 is configured to combine the outputs or triggers of the plurality of predictive models 104a-104n to generate an alert based on one or more rules and the model scores received from the plurality of predictive models 104a-104n using the model scores comparison component 212, the rules component 214 and the conflict evaluation component 216.

The model scores comparison component 212 may comprise suitable logic, interfaces, and/or code that may be configured to compare the model scores received from the plurality of predictive models 104a-104n with one or more model-specific thresholds at a given time (a timepoint or window), to obtain a set of alert trigger signals.

The rules component 214 may comprise suitable logic, interfaces, and/or code that may configured to provide one or more rules to aid decision-making of the conflict evaluation component 216 to finally generate an alert. The rules may include, but are not limited to, single timepoint-based rules, a prioritization matrix and windowing-based rules.

The conflict evaluation component 216 may comprise suitable logic, interfaces, and/or code that may configured to determine if one or more alert trigger signals in the set of alert trigger signals is conflicting with one or more other alert trigger signals in the set of alert trigger signals based on the one or more rules. In response to a determination that there is a conflict between any of the alert trigger signals, the conflict evaluation component 216 signals to the alert generator 218 to suppress the generation of any alert.

In accordance with an embodiment, the processing system 206 is configured to combine the outputs of the plurality of predictive models 104a-104n to generate an alert based on single timepoint-based rules. In this particular embodiment, in response to a determination by the conflict evaluation component 216 that alert trigger signals do not conflict with each other, the conflict evaluation component 216 selects at each timepoint, an alert trigger signal from the set of alert trigger signals based on a severity level of the alert trigger signal, and signals to the alert generator 218 to generate the alert based on the selected alert trigger signal.

For example, for a two-model predictive system including an any-disease predictive model and a severe-disease predictive model, in one instance, upon determining by the conflict evaluation component 216, that the any-disease predictive model and the severe-disease predictive model both generate alert trigger signals, the conflict evaluation component 216 signals to the alert generator 218 to issue an alert corresponding to a most-severe alert trigger signal.

In another instance, upon determining by the conflict evaluation component 216, that the severe-disease predictive model generates an alert trigger signal but the any-disease predictive model does not generate an alert trigger signal, the conflict evaluation component 216 signals to the alert generator 218 to suppress issuance of an alert.

An algorithm for generating a single alert using single timepoint-based-rules is illustrated as follows:

Consider the following variable definitions,

Timepoints: $t \in \{1, ..., T\}$

Models: $m \in \{1, ..., M\}$

Scores for each model at each timepoint: $s_t^m$

Thresholds for each model: $\tau^m$

At each timepoint, compare the scores for all models with the model-specific thresholds to get a trigger:

$$I(s_t^m > \tau^m) = \begin{cases} 0 & if \quad s_t^m \leq \tau^m \\ 1 & if \quad s_t^m > \tau^m \end{cases}$$

Examine the set of all triggers to determine a single alert:

$$\{I(s_t^1 > \tau^1), ..., I(s_t^M > \tau^M)\}$$

If any of the triggers are contradictory (for example, if a severe-disease model triggers but the any-disease model does not), an alert is not issued. Which sets contain contradictory triggers becomes more complicated as the number of models (M) gets larger.

If none of the models trigger, an alert is not issued. Otherwise, an alert is issued corresponding to the most-severe model trigger.

For example, if both the any-disease and the severe-disease models trigger, an alert is issued for severe disease.

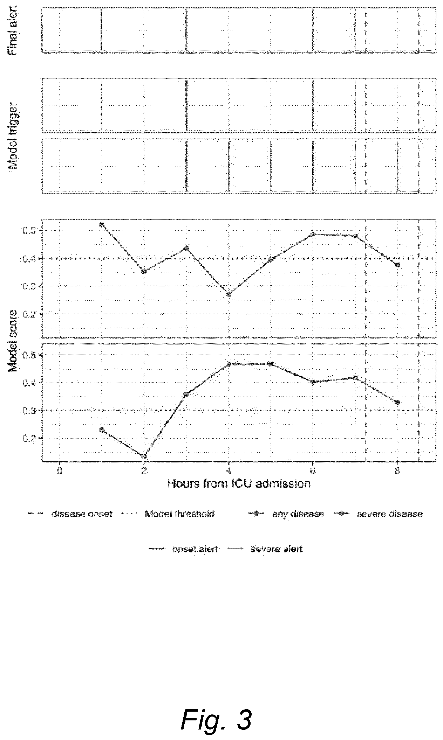

Joint alerting using single timepoint-based rules is further illustrated in conjunction with FIG. 3.

In accordance with another embodiment, the processing system 206 is configured to combine the outputs of the plurality of predictive models 104a-104n to generate an alert based on a prioritization matrix. The prioritization matrix is an M x M matrix of weights given to different combinations of the plurality of predictive models 104a-104n.

The processing system 206 is configured to generate an alert weight vector comprising alert weights corresponding to each predictive model by multiplying an alert trigger signal vector by the prioritization matrix. The alert trigger signal vector comprises alert trigger signals from the plurality of predictive models.

In one instance, the conflict evaluation component 216 is configured to signal to the alert generator 218 to suppress generation of the alert if at least one of the alert weights in the alert weight vector is less than zero. In another instance, the conflict evaluation component 216 is configured to signal to the alert generator 218 to generate an alert corresponding to a predictive model with a maximum alert weight in the alert weight vector.

For example, for a two-model predictive system comprising an any-disease predictive model and a moderate/severe-disease predictive model, in one instance, upon determining by the conflict evaluation component 216 that the any-disease predictive model generates an alert trigger signal and the moderate/severe-disease predictive model does not generate any alert trigger signal, the conflict evaluation component 216 signals to the alert generator 218 to issue an alert corresponding to the any-disease predictive model.

In another instance, upon determining by the conflict evaluation component 216 that the any-disease predictive model does not generate any alert trigger signal and the moderate/severe-disease predictive model generates an alert trigger signal, the conflict evaluation component 216 signals to the alert generator 218 to suppress issuance of an alert due to the contradiction in the determination.

In yet another instance, upon determining by the conflict evaluation component 216 that the any-disease predictive model and the moderate/severe-disease predictive model both generate alert trigger signals, the conflict evaluation component 216 signals to the alert generator 218 to issue an alert corresponding to the moderate/severe-disease predictive model.

An algorithm for generating a single alert using a prioritization matrix is illustrated as follows:

A prioritization matrix is an M x M matrix of weights given to different combinations of models:

$$P = \begin{bmatrix} w_{11} & \cdots & w_{1M} \\ \vdots & \ddots & \vdots \\ w_{M1} & \cdots & w_{MM} \end{bmatrix}$$

If $w_{ij} < 0$, then there is a contradiction if model i alerts but model j does not. For example, the prioritization matrix for a two-model system (one detecting any disease and another detecting severe disease) could be:

To determine which, if any, alert is generated, the trigger vector is multiplied by the prioritization matrix: this generates an alert weight vector as follows:

$$[I(s_t^1 > \tau^1) \quad ... \quad I(s_t^M > \tau^M)] \begin{bmatrix} w_{11} & \cdots & w_{1M} \\ \vdots & \ddots & \vdots \\ w_{M1} & \cdots & w_{MM} \end{bmatrix} = \begin{bmatrix} a_t^1 \\ \vdots \\ a_t^M \end{bmatrix}$$

An alert is not generated if any $a_t^i$ is < 0

The final alert is generated corresponding to the model with the maximum $a_t^i$.

For example, if model 1 predicts any disease, and model 2 predicts moderate/severe disease,

$$P = \begin{bmatrix} 2 & 1 \\ -2 & 2 \end{bmatrix}$$

Prioritization matrix

If model 1 triggers but model 2 does not, the resulting alert vector is:
$$[1 \quad 0] \begin{bmatrix} 2 & 1 \\ -2 & 2 \end{bmatrix} = [2 \quad 1]$$

In this case, an alert would be generated for model 1.

On other hand, if model 2 triggers but model 1 does not, the resulting alert vector is:
$$[0 \quad 1] \begin{bmatrix} 2 & 1 \\ -2 & 2 \end{bmatrix} = [-2 \quad 1]$$

In this case, no alert is generated because of the contradiction.

$$[1 \quad 1] \begin{bmatrix} 2 & 1 \\ -2 & 2 \end{bmatrix} = [0 \quad 3]$$

If both models trigger, the resulting alert vector is:

In this case, an alert for model 2 (the severe model) would be generated.

In accordance with yet another embodiment, the processing system 206 is configured to combine the outputs of the plurality of predictive models 104a-104n to generate an alert based on windowing-based rules. In response to a determination by the conflict evaluation component 216 that the alert trigger signals do not conflict with each other, the conflict evaluation system 216 selects at each window, an alert trigger signal from the set of alert trigger signals based on a severity level of the alert trigger signal, and signals to the alert generator 218 to generate an alert based on the selected alert trigger signal.

For example, for a two-model predictive system comprising an any-disease predictive model and a severe-disease predictive model, in one instance, upon determining by the conflict evaluation component 216 if the any-disease predictive model and the severe-disease predictive model both generate alert trigger signals, the conflict evaluation component 216 signals to the alert generator 218 to issue an alert corresponding to a most-severe alert trigger signal.

In another instance, upon determining by the conflict evaluation component 216 that the severe-disease predictive model generates an alert trigger signal but the any-disease predictive model does not generate an alert trigger signal, the conflict evaluation component 216 signals to the alert generator 218 to suppress issuance of an alert.

An algorithm for generating a single alert using windowing-based rules is illustrated as follows:

The duration of the window can be defined as w (for example, w could be one hour). This involves re-defining a model 'trigger' at each timepoint.

Use

$$I(s_t^m > \tau^m) = \begin{cases} 0 & if \quad s_{t'}^m \leq \tau^m \ for \ all \ t - w \leq t' \leq t \\ 1 & if \quad s_{t'}^m > \tau^m \ for \ any \ t - w \leq t' \leq t \end{cases}$$

It is also possible for the windows to be algorithm-specific ($w_m$ rather than w). The process of combining the triggers would then proceed in the same manner as discussed in the embodiment for single timepoint-based rules.

FIG. 3 is a schematic illustrating how scores from two models can be combined using the proposed rules in accordance with an exemplary embodiment of the disclosure. Referring to FIG. 3, there is shown a bottom panel representing scores for two separate models, one predicting any disease onset, and the other predicting severe disease onset. At each timepoint, the model scores are compared with the model-specific thresholds to generate 'triggers' (middle panel). Finally, the triggers from each model are combined to generate a single alert (top panel).

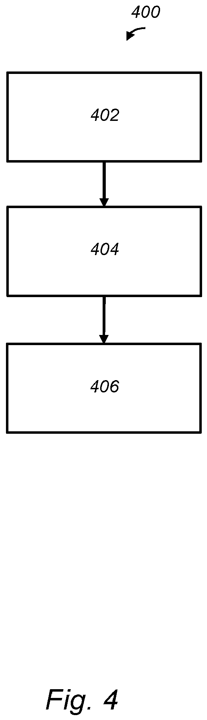

FIG. 4 is a flowchart illustrating a method for generating an alert in accordance with an exemplary embodiment of the disclosure. Referring to FIG. 4, there is shown a flowchart of a method 400 for generating an alert using the alert generation apparatus 102.

At 402, receive outputs corresponding to each predictive model of a plurality of predictive models. The receiving component 210 receives outputs from the plurality of predictive models 104a-104n.

At 404, receive model scores corresponding to each predictive model of the plurality of predictive models. The receiving component 210 receives the model scores corresponding to each predictive model of the plurality of predictive models 104a-104n.

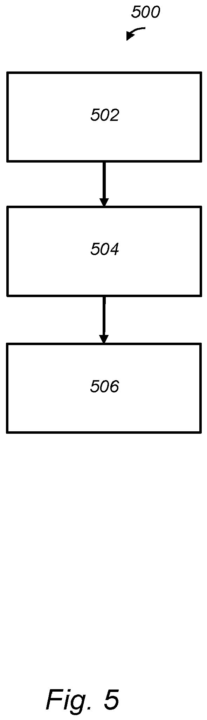

At 406, combine the outputs of the plurality of predictive models to generate an alert at a given time based on one or more rules and the model scores received for each predictive model of the plurality of predictive models. The processing system 206 of the alert generation apparatus 102 is configured to combine the outputs of the of the plurality of predictive models 104a-104n to generate a single alert at a given time (e.g. a timepoint or a window) based on one or more rules and the model scores received for each predictive model of the plurality of predictive models 104a-104n. The one or more rules may include, but are not limited to, single timepoint-based rules, prioritization matrix and windowing-based rules. This step is further illustrated in conjunction with FIG. 5.

FIG. 5 is a flowchart of a method for combining the outputs of the plurality of predictive models to generate an alert accordance with an exemplary embodiment of the disclosure. Referring to FIG. 5, there is shown a flowchart of a method 500 for combining the outputs of the plurality of predictive models 104a-104n to generate an alert using the alert generation apparatus 102.

At 502, compare the model scores received for each predictive model of the plurality of predictive models with one or more model-specific thresholds at a given time, to obtain a set of alert trigger signals. The model scores comparison component 214 compares the model scores received for each predictive model of the plurality of predictive models 104a-104n with one or more model-specific thresholds at a given time (a timepoint or window), to obtain a set of alert trigger signals.

At 504, determine if at least one alert trigger signal in the set of alert trigger signals is conflicting with at least one other alert trigger signal in the set of alert trigger signals based on the one or more rules. The conflict evaluation component 216 determines if at least one alert trigger signal in the set of alert trigger signals is conflicting with at least one other alert trigger signal in the set of alert trigger signals based on the one or more rules.

At 506, suppress generation of the alert in response to a determination that the at least one alert trigger signal conflicts with the at least one other alert trigger signal. The conflict evaluation component 216 signals to the alert generator 218 to suppress generation of the alert in response to a determination that the at least one alert trigger signal conflicts with the at least one other alert trigger signal.

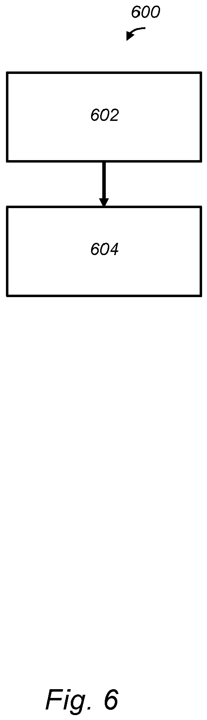

FIG. 6 is a flowchart illustrating a method for combining outputs of predictive models using single timepoint-based rules in accordance with an exemplary embodiment of the disclosure. Referring to FIG. 6, there is shown a flowchart of a method 600 for combining outputs of predictive models using single timepoint-based rules in a two-model predictive system including an any-disease predictive model and a severe-disease predictive model.

At 602, upon determining if the any-disease predictive model and the severe-disease predictive model both generate alert trigger signals, issue an alert corresponding to a most-severe alert trigger signal.

At 604, upon determining if the severe-disease predictive model generates an alert trigger signal but the any-disease predictive model does not generate an alert trigger signal, suppress issuance of an alert.

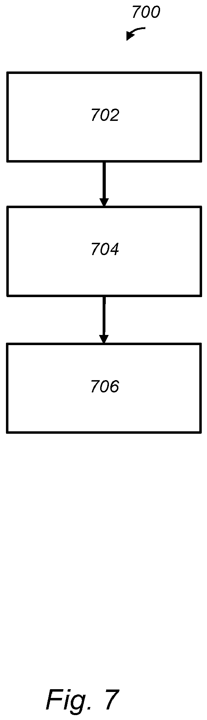

FIG. 7 is a flowchart illustrating a method for combining outputs of predictive models using a prioritization matrix in accordance with an exemplary embodiment of the disclosure. Referring to FIG. 7, there is shown a flowchart of a method 700 for combining outputs of predictive models using a prioritization matrix in a two-model predictive system including an any-disease predictive model and moderate/severe-disease predictive model.

At 702, upon determining if the any-disease predictive model generates an alert trigger signal and the moderate/severe-disease predictive model does not generate any alert trigger signal, issue an alert corresponding to the any-disease predictive model.

At 704, upon determining if the any-disease predictive model does not generate any alert trigger signal and the moderate/severe-disease predictive model generates an alert trigger signal, suppress issuance of an alert due to the contradiction in the determination.

At 706, upon determining if the any-disease predictive model and the moderate/severe-disease predictive model both generate alert trigger signals, issue an alert corresponding to the moderate/severe-disease predictive model.

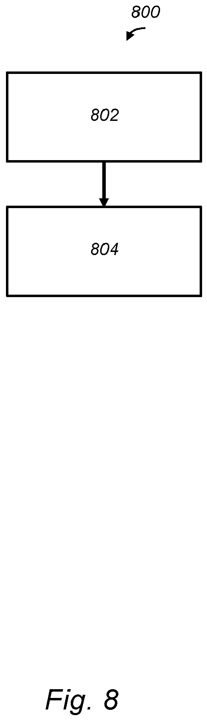

FIG. 8 is a flowchart illustrating a method for combining outputs of predictive models using windowing-based rules in accordance with an exemplary embodiment of the disclosure. Referring to FIG. 8, there is shown a flowchart of a method 800 for combining outputs of predictive models using windowing-based rules in a two-model predictive system including an any-disease predictive model and a severe-disease predictive model.

At 802, upon determining if the any-disease predictive model and the severe-disease predictive model both generate alert trigger signals, issue an alert corresponding to a most-severe alert trigger signal.

At 804, upon determining if the severe-disease predictive model generates an alert trigger signal but the any-disease predictive model does not generate an alert trigger signal, suppress issuance of an alert.

Many different ways of executing the method described above are possible, as will be apparent to a person skilled in the art. For example, the order of the steps can be performed in the shown order, but the order of the steps can be varied, or some steps may be executed in parallel. Moreover, in between steps other method steps may be inserted. The inserted steps may represent refinements of the method such as described herein or may be unrelated to the method. For example, some steps may be executed, at least partially, in parallel. Moreover, a given step may not have finished completely before a next step is started.

Embodiments of the method may be executed using software, which comprises instructions for causing a processor system to perform methods 400, 500, 600, 700 and 800. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

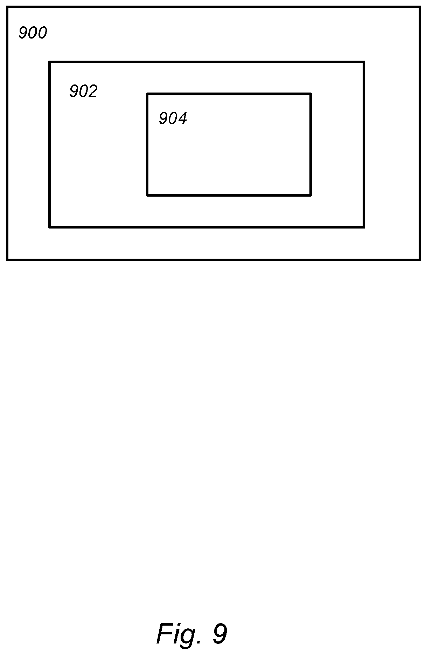

FIG. 9 is a block diagram illustrating an example computer program product in accordance with an exemplary embodiment of the disclosure. Referring to FIG. 9, there is shown a computer program product 900 that includes a machine-readable storage 902 that may also include logic 904. In some implementations, the machine-readable storage 902 may be implemented as a non-transitory machine-readable storage. In some implementations the logic 904 may be implemented as machine-readable instructions, such as software, for example. In an embodiment, the logic 904, when executed, implements one or more aspects of the method 400 (FIG. 4), the method 500 (FIG. 5), the method 600 (FIG. 6), the method 700 (FIG. 7), and the method 800 (FIG. 8) and/or realize the alert generation apparatus 102 (FIG. 2), already discussed.

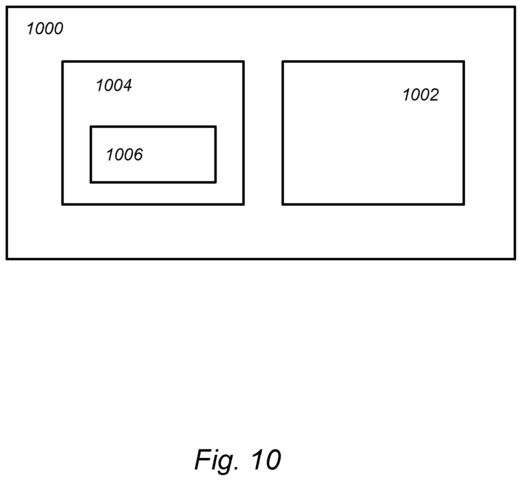

FIG. 10 is a diagram illustrating an example of the alert generation apparatus in accordance with an exemplary embodiment of the disclosure. Referring to FIG. 10, in the illustrated example, the alert generation apparatus 102 may include a processor 1002 and a memory 1004 communicatively coupled to the processor 1002. The memory 1004 may include logic 1006 as a set of instructions. In some implementations, the logic 1006 may be implemented as software.

10

In an embodiment, the logic 1006, when executed by the processor 1002, implements one or more aspects of the method 400 (FIG. 4), the method 500 (FIG. 5), the method 600 (FIG. 6), the method 700 (FIG. 7), and the method 800 (FIG. 8), and/or realize the alert generation apparatus 102 (FIG. 2), already discussed.

In some implementations, the processor 1002 may include a general purpose controller, a special purpose controller, a storage controller, a storage manager, a memory controller, a microcontroller, a general purpose processor, a special purpose processor, a central processor unit (CPU), the like, and/or combinations thereof.

Further, implementations may include distributed processing, component/object distributed processing, parallel processing, the like, and/or combinations thereof. For example, virtual computer system processing may implement one or more of the methods or functionalities as described herein, and the processor 1002 described herein may be used to support such virtual processing.

In some examples, the memory 1004 is an example of a computer-readable storage medium. For example, memory 1004 may be any memory which is accessible to the processor 1002, including, but not limited to RAM memory, registers, and register files, the like, and/or combinations thereof. References to "computer memory" or "memory" should be interpreted as possibly being multiple memories. The memory may for instance be multiple memories within the same computer system. The memory may also be multiple memories distributed amongst multiple computer systems or computing devices.

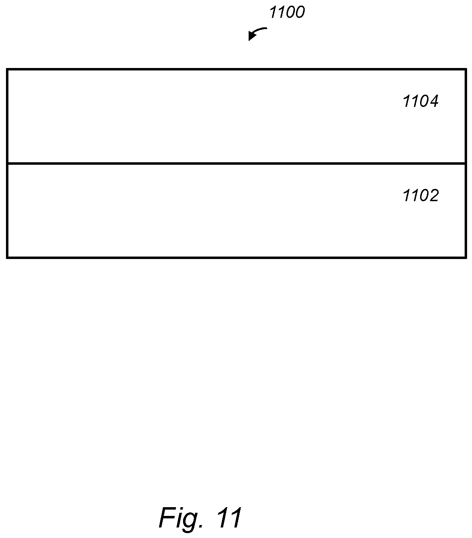

FIG. 11 is a diagram illustrating a semiconductor apparatus in accordance with an exemplary embodiment of the disclosure. The illustrated semiconductor apparatus 1100 (e.g., chip and/or package) includes one or more substrates 1102 (e.g., silicon, sapphire, or gallium arsenide) and logic 1104 (e.g., configurable logic and/or fixed-functionality hardware logic) coupled to the substrate(s) 1102. In an embodiment, the logic 1104 implements one or more aspects of the method 400 (FIG. 4), the method 500 (FIG. 5), the method 600 (FIG. 6), the method 700 (FIG. 7), and the method 800 (FIG. 8), and/or realize the alert generation apparatus 102 (FIG. 2), already discussed.

In some implementations, logic 1104 may include transistor array and/or other integrated circuit/IC components. For example, configurable logic and/or fixed-functionality hardware logic implementations of the logic 1104 may include configurable logic such as, for example, programmable logic arrays (PLAs), field programmable gate arrays (FPGAs), complex programmable logic devices (CPLDs), or fixed-functionality logic hardware using circuit technology such as, for example, application specific integrated circuit (ASIC), complementary metal oxide semiconductor (CMOS) or transistor-transistor logic (TTL) technology, the like, and/or combinations thereof.

All definitions, as defined and used herein, should be understood to control over dictionary definitions, definitions in documents incorporated by reference, and/or ordinary meanings of the defined terms.

The subject matter described herein sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. The term "coupled" may be used herein to refer to any type of relationship, direct or indirect, between the components in question, and may apply to electrical, mechanical, fluid, optical, electromagnetic, electromechanical, or other connections. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components.

In the claims, as well as in the specification above, the terms "first", "second", etc. may be used herein only to facilitate discussion, and carry no particular temporal or chronological significance unless otherwise indicated.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The indefinite articles "a" and "an," as used herein in the specification and in the claims, unless clearly indicated to the contrary, should be understood to mean "at least one."

As used herein, the term "or" or "and/or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used in this application and in the claims, a list of items joined by the term "one or more of' may mean any combination of the listed terms. For example, the phrases "one or more of A, B or C" may mean A; B; C; A and B; A and C; B and C; or A, B and C.

As is described above in greater detail, one or more processor, other unit, the like, and/or combinations thereof may fulfill the functions of several items recited in the claims.

As is described above in greater detail, a computer program may be stored/distributed on a suitable medium, such as an optical storage medium or a solid-state medium supplied together with or as part of other hardware, but may also be distributed in other forms, such as via the Internet or other wired or wireless telecommunication systems.

It should also be understood that, unless clearly indicated to the contrary, in any methods discussed herein that include more than one step or act, the order of the steps or acts of the method is not necessarily limited to the order in which the steps or acts of the method are recited. Further, such methods may include additional or alternative steps or acts. As used in the claims, the mere fact that certain measures are recited in mutually different dependent claims does not indicate that a combination of these measured cannot be used to advantage.

Those skilled in the art will appreciate from the foregoing description that the broad techniques of the embodiments of the present disclosure can be implemented in a variety of forms. Therefore, while the embodiments of this disclosure have been described in connection with particular examples thereof, the true scope of the embodiments of the disclosure should not be so limited since other modifications will become apparent to the skilled practitioner upon a study of the drawings, specification, and following claims.

**Claims**

1. An apparatus for generating an alert, comprising:

   a user interface; and
   a framework communicatively coupled to the user interface, the framework comprising a processing system configured to:

   receive outputs corresponding to each predictive model of a plurality of predictive models;
   receive model scores corresponding to each predictive model of the plurality of predictive models;
   combine the outputs of the plurality of predictive models to generate an alert at a given time based on one or more rules and the model scores received for each predictive model of the plurality of predictive models, wherein the processing system is further configured to:

   compare the model scores received for each predictive model of the plurality of predictive models with one or more model-specific thresholds at the given time, to obtain a set of alert trigger signals;
   determine if at least one alert trigger signal in the set of alert trigger signals is conflicting with at least one other alert trigger signal in the set of alert trigger signals based on the one or more rules; and
   suppress generation of the alert in response to a determination that the at least one alert trigger signal conflicts with the at least one other alert trigger signal.

2. The apparatus of claim 1, wherein the given time is at least one of a timepoint and a time window.

3. The apparatus of claim 1, wherein the plurality of predictive models are trained for predicting potential risk of occurrence of a health condition at varying levels of severity in clinical decision support (CDS) systems.

4. The apparatus of claim 1, wherein the processing system is configured to combine the outputs of the plurality of predictive models to generate an alert based on single timepoint-based rules, wherein in response to a determination that the at least one alert trigger signal does not conflict with the at least one other alert trigger signal, the processing system is configured to:

   select at each timepoint, an alert trigger signal from the set of alert trigger signals based on a severity level of the alert trigger signal; and
   generate the alert based on the selected alert trigger signal.

5. The apparatus of claim 4, wherein for a two-model predictive system including an any-disease predictive model and a severe-disease predictive model,

upon determining if the any-disease predictive model and the severe-disease predictive model both generate alert trigger signals, the processing system is configured to issue an alert corresponding to a most-severe alert trigger signal; and
upon determining if the severe-disease predictive model generates an alert trigger signal but the any-disease predictive model does not generate an alert trigger signal, the processing system is configured to suppress issuance of an alert.

6. The apparatus of claim 1, wherein the processing system is configured to combine the outputs of the plurality of predictive models to generate an alert based on a prioritization matrix, wherein the prioritization matrix is an M x M matrix of weights given to different combinations of the plurality of predictive models.

7. The apparatus of claim 6, wherein the processing system is configured to generate an alert weight vector comprising alert weights corresponding to each predictive model of the plurality of predictive models by multiplying an alert trigger signal vector by the prioritization matrix, wherein the alert trigger signal vector comprises alert trigger signals from the plurality of predictive models.

8. The apparatus of claim 7, wherein the processing system is configured to suppress generation of the alert if at least one of the alert weights in the alert weight vector is less than zero.

9. The apparatus of claim 7, wherein the processing system is configured to generate an alert corresponding to a predictive model with a maximum alert weight in the alert weight vector.

10. The apparatus of claims 6 to 9, wherein for a two-model predictive system comprising an any-disease predictive model and a moderate/severe-disease predictive model,

upon determining if the any-disease predictive model generates an alert trigger signal and the moderate/severe-disease predictive model does not generate any alert trigger signal, the processing system is configured to issue an alert corresponding to the any-disease predictive model;
upon determining if the any-disease predictive model does not generate any alert trigger signal and the moderate/severe-disease predictive model generates an alert trigger signal, the processing system is configured to suppress issuance of an alert due to the contradiction in the determination; and
upon determining if the any-disease predictive model and the moderate/severe-disease predictive model both generate alert trigger signals, the processing system is configured to issue an alert corresponding to the moderate/severe-disease predictive model.

11. The apparatus of claim 1, wherein the processing system is configured to combine the outputs of the plurality of predictive models to generate an alert based on windowing-based rules, wherein in response to a determination that the at least one alert trigger signal does not conflict with the at least one other alert trigger signal, the processing system is configured to:

select at each window, an alert trigger signal from the set of alert trigger signals based on a severity level of the alert trigger signal; and
generate the alert based on the selected alert trigger signal.

12. The apparatus of claim 11, wherein for a two-model predictive system comprising an any-disease predictive model and a severe-disease predictive model,

upon determining if the any-disease predictive model and the severe-disease predictive model both generate alert trigger signals, the processing system is configured to issue an alert corresponding to a most-severe alert trigger signal; and
upon determining if the severe-disease predictive model generates an alert trigger signal but the any-disease predictive model does not generate an alert trigger signal, the processing system is configured to suppress issuance of an alert.

13. A method for generating an alert, comprising:

EP 4 261 842 A1

receiving outputs corresponding to each predictive model of a plurality of predictive models;
receiving model scores corresponding to each predictive model of the plurality of predictive models;
combining the outputs of the plurality of predictive models to generate an alert at a given time based on one or more rules and the model scores received for each predictive model of the plurality of predictive models, wherein the combining further comprises:

comparing the model scores received for each predictive model of the plurality of predictive models with one or more model-specific thresholds at the given time, to obtain a set of alert trigger signals;
determining if at least one alert trigger signal in the set of alert trigger signals is conflicting with at least one other alert trigger signal in the set of alert trigger signals based on the one or more rules; and
suppressing generation of the alert in response to a determination that the at least one alert trigger signal conflicts with the at least one other alert trigger signal.

14. The method of claim 13, wherein the combining comprises combining the outputs of the plurality of predictive models to generate an alert based on single timepoint-based rules, wherein in response to a determination that the at least one alert trigger signal does not conflict with the at least one other alert trigger signal,

selecting at each timepoint, an alert trigger signal from the set of alert trigger signals based on a severity level of the alert trigger signal; and
generating the alert based on the selected alert trigger signal.

15. The method of claim 14, wherein for a two-model predictive system including an any-disease predictive model and a severe-disease predictive model,

upon determining if the any-disease predictive model and the severe-disease predictive model both generate alert trigger signals, issuing an alert corresponding to a most-severe alert trigger signal; and
upon determining if the severe-disease predictive model generates an alert trigger signal but the any-disease predictive model does not generate an alert trigger signal, suppressing issuance of an alert.

*Fig. 1*

*102*

*200*

| | | |
|---|---|---|
| | *208* | *210* |
| *202* | | *212* |
| *204* | | *214* |
| *206* | | *216* |
| | | *218* |

## Fig. 2

Fig. 3

400

402

404

406

Fig. 4

*500*

```
┌─────────────────────┐
│                     │
│        502          │
│                     │
└─────────────────────┘
           │
           ▼
┌─────────────────────┐
│                     │
│        504          │
│                     │
└─────────────────────┘
           │
           ▼
┌─────────────────────┐
│                     │
│        506          │
│                     │
└─────────────────────┘
```

*Fig. 5*

*600*

```
┌─────────────────────────┐
│                         │
│          602            │
│                         │
└─────────────────────────┘
             │
             ▼
┌─────────────────────────┐
│                         │
│          604            │
│                         │
└─────────────────────────┘
```

*Fig. 6*

*700*

```
┌─────────────────────┐
│                     │
│         702         │
│                     │
└─────────────────────┘
           │
           ▼
┌─────────────────────┐
│                     │
│         704         │
│                     │
└─────────────────────┘
           │
           ▼
┌─────────────────────┐
│                     │
│         706         │
│                     │
└─────────────────────┘
```

*Fig. 7*

*800*

```
          ┌──────────────────────┐
          │                      │
          │         802          │
          │                      │
          └──────────┬───────────┘
                     │
                     ▼
          ┌──────────────────────┐
          │                      │
          │         804          │
          │                      │
          └──────────────────────┘
```

*Fig. 8*

900

902

904

Fig. 9

1000

1004

1006

1002

Fig. 10

*1100*

1104

1102

*Fig. 11*

Europäisches
Patentamt

European
Patent Office

Office européen
des brevets

# EUROPEAN SEARCH REPORT

Application Number

**EP 22 18 7566**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/206797 A1 (SAMIEE KAVEH [FI]) 26 July 2018 (2018-07-26) * paragraphs [0011] – [0039] * ----- | 1-15 | INV. G16H50/20 G16H50/30 |
| A | US 2019/130730 A1 (BOYER ROBERT T [US]) 2 May 2019 (2019-05-02) * paragraph [0047] * ----- | 1-15 | |

TECHNICAL FIELDS SEARCHED (IPC)

G16H

The present search report has been drawn up for all claims

| Place of search | Date of completion of the search | Examiner |
|---|---|---|
| Munich | 11 May 2023 | Reinbold, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 7566

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2023

| Patent document cited in search report | Publication date | Patent family member(s) | | Publication date |
|---|---|---|---|---|
| US 2018206797 A1 | 26-07-2018 | NONE | | |
| US 2019130730 A1 | 02-05-2019 | US | 2019130730 A1 | 02-05-2019 |
| | | WO | 2019089084 A1 | 09-05-2019 |

For more details about this annex : see Official Journal of the European Patent Office, No. 12/82